# EUROPÄISCHE PATENTANMELDUNG

(11) **EP 0 105 102 A1**
(43) Veröffentlichungstag der Anmeldung: **11.04.1984**
(21) Anmeldenummer: 83106817.6
(22) Anmeldetag: 12.07.1983
(51) Int. Cl.: C07C 103/52, A61K 31/435, C07D 453/06

(54) **Neue Derivate der 2-Aza-bicyclo(2.2.2)octan-3-carbonsäure, Verfahren zu ihrer Herstellung, diese enthaltende Mittel und deren Verwendung sowie 2-Aza-bicyclo(2.2.2)octan-3-carbonsäure als Zwischenstufe und Verfahren zu deren Herstellung**

(30) Priorität: 20.07.1982 DE 3227055
(71) Anmelder: HOECHST AKTIENGESELLSCHAFT, 65926 Frankfurt am Main (DE)
(72) Erfinder: Henning, Rainer, Dr., D-6000 Frankfurt am Main 71 (DE); Urbach, Hansjörg, Dr., D-6242 Kronberg/Taunus (DE); Becker, Reinhard, Dr., D-6200 Wiesbaden (DE)

(57) **Zusammenfassung**

Die Erfindung betrifft neue Derivate der 2-Aza- bicyclo[2,2,2]-octan-3-carbonsäure der Formel I 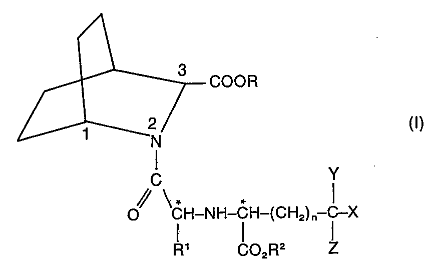 in der n 0 oder 2, R Wasserstoff, Alkyl oder Aralkyl, R' Wasserstoff oder gegebenenfalls durch Amino, Acylamino oder Benzoylamino substituiertes Alkyl, Alkenyl, Cycloalkyl, Cycloalkenyl, Cycloalkylalkyl, Aryl oder teilhydriertes Aryl, das jeweils durch Alkyl, Alkoxy oder Halogen substituiert sein kann, Aralkyl oder Aroylalkyl die beide wie vorstehend definiert, im Arylrest substituiert sein können, ein S- oder O- und/oder N-Heterocyclen-Rest oder eine Seitenkette einer Aminosäure, R² Wasserstoff, Alkyl, Alkenyl oder Aralkyl, Y Wasserstoff oder Hydroxy, Z Wasserstoff oder Y und Z zusammen Sauerstoff und X Alkyl, Alkenyl, Cycloalkyl, Aryl, das durch Alkyl, Alkoxy, Hydroxy, Halogen, Nitro, Amino, Alkylamino, Dialkyl-amino und/oder Methylendioxy mono-, di-oder trisubstituiert sein kann oder 3-Indolyl bedeuten, Verfahren zu ihrer Herstellung, diese enthaltende Mittel und deren Verwendung sowie 2-Aza-bicyclo[2,2,2]-octan-3-carbonsäure als Zwischenstufe und Verfahren zu deren Herstellung.

## Beschreibung

Die Erfindung betrifft neue Derivate der 2-Aza-bicyclo [2.2.2]-octan-3-carbonsäure der Formel I 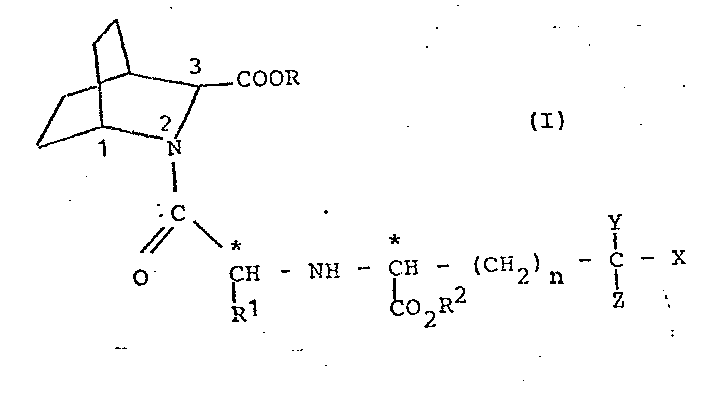 in der
n. = O oder 1,
R = Wasserstoff, (C₁ bis C₆)-Alkyl oder Aralkyl mit 7 bis 9 C-Atomen,
R¹ = Wasserstoff oder (C₁ bis C₆)-Alkyl, das gegebenenfalls durch Amino, (C₁ bis C ₄)-Acylamino vorzugsweise
(C₁ bis C₄)-Alkanoylamino oder Benzoylamino substituiert sein kann, (C₂ bis C₆)- Alkenyl, (C₅ bis C₉)-Cycloaihyl, (C₅ bis C₉)-Cycloalkeyl, (C₅ bis C₇)-Cycloalkyl-(C₁ bis C₄)-alkyl, (C₆ bis C₁₀)-Aryl oder teilhydriertes (C₆ bis C₁₀)-Aryl, das jeweils durch (C ₁ bis C₄)-Alkyl, (C₁oder C₂)-Alkoxy oder Halogen substituiert sein kann, (C₆ bis C₁₀)-Aryl-(C₁ bis C₄)-alkyl oder (C₇ bis C₁₁ )-Aroyl-(C₁ bis C₄)-alkyl, die beide wie vorstehend dofiniert, im Arylrest substituiert sein können, ein mono- bzw. bicyclischer Heterocyclen- Rest mit 5 bis 7 bzw. 8 bis 10 Ringatoman, wovon 1 bis 2 Ringatome Schwefel- oder Sauerstoffatome und/oder wovon 1 bis 4 Ringatome Stickstoffatome darstellen, oder eine gegebenenfalls geschützte Seitenkette einer natürlich vorkommenden α-Aminosäure,
_{R}²= Wasserstoff, (C bis C₆)-Alkyl, (C₂ bis C₆)-Alkenyl oder (C₆ bis C₁₀)-Aryl-(C₁ bis C₄)-alkyl,
Y = Wasserstoff oder Hydroxy,
Z = Wasserstoff oder
Y und Z = zusammen Sauerstoff und
X = (C₁ bis C₆)-Alkyl, (C₂ bis C₆)-Alkenyl, (C₅ bis C₉)-Cycloalkyl, (C₆ bis C₁₀)-Aryl, das durch (C₁ bis C₄)-Alkyl, (C₁ bis C₄)-Alkoxy, Hydroxy, Halogen, Nitro, Amino, (C₁ bis C₄)-Alk^{y}lamino, Di-(C₁ bis C₄)alkylamino und/oder Methylendioxy mono-, di- oder trisubstituiert sein kann oder 3-Indolyl bedeuten,

sowie deren physiologisch unbedenkliche Salze.

Als Salze kommen insbesondere Alkali- und Erdalkalisalze, Salze mit physiologisch verträglichen Aminen und Salze mit anorganischen oder organischen Säuren wie z.B. HC1, HBr, H₂SO₄, Maleinsäure, Furmarsäure in Frage.

Unter Aryl ist hier wie im folgenden vorzugsweise gegebenenfalls substituiertes Phenyl oder Naphthyl, unter Aroyl vorzugsweise gegebenenfalls substituiertes Benzoyl oder Naphthoyl, zu verstehen. Alkyl kann geradkettig oder verzweigt sein.

Unter einem mono- bzw. bicyclischen Heterocylen-Rest mit 5 bis 7 bzw. 8 bis 10 Ringatomen, wovon 1 bis 4 Ringatome Stickstoffatome und/oder wovon 1 bis 4 Ringatome Stickstoffatome darstellen, wird beispielsweise Thienyl, Benzo/b/thienyl, Furyl, Pyranyl, Benzofuryl, Pyrrolyl, Imidazolyl, Pyrazolyl, Pyridyl, Pyrimidinyl, Pyridazinyl, Indazolyl, Isoindolyl, Indolyl, Purinyl, Chinolizinyl, Isochinolinyl, Phthalazinyl, Naphthyridinyl, Chinoxalinyl, Chinazolyl, Cinnolinyl, Pteridinyl, Oxazolyl, Isoxazolyl, Thiazolyl oder Isothiazolyl verstanden. Diese Reste können auch teilweise oder vollständig hydriert sein.

Natürlich vorkommende α -Aminosäuren sind z.B. Ala, Val, Len, Ile, Phe, Ser, Thr, Lys, Hyl, Arg, Asp, Asn, Glu, Gln, Cys, Met, Tyr, Pro, Hyp, Trp, His, Orn und Cit.

Falls R für eine geschützte Seitenkette einer natürlich vorkommenden α -Aminosäure steht, wie z.B. geschütztes Ser, Thr, Asp, Asn, Glu, Gln, Arg, Lys, Hyl, Cyl, Orn, Cit, Tyr, Trp, His oder Hyp, sind als Schutzgruppen die in der Peptidchemie üblichen Gruppen bevorzugt (vgl. Houben-Weyl, Bd. XV/1 und XV 2 ). Im Falle, daß R¹ die geschützte Lysin-Seitenkette bedeutet, werden die bekannten Amino-Schutzgruppen, insbesondere aber (C₁ - C₆)-Alkanoyl bovorzugt. Bevorzugte O-Schutzgruppen für Tyrosin sind Methyl oder Ethyl.

Verbindungen der Formel I besitzen chirale C-Atome in der Position C-3, sowie in den mit einem Stern markierten C-Atomen der Seitenketten. Sowohl die R- als auch die S-Konfigurationen an allen Zentren sind Gegenstand der Erfindung. Die Verbindungen der Formel I können daher als optische Isomere, als Diasteromere, als Racemate oder als Gemische derselben vorliegen. Bevorzugt sind jedoch die Verbindungen der Formel I, in denen das C-Atom 3 im bicyclischen Ringsystem, sowie die mit einem Stern (^{*}) .markierten C-Atome der Seitenkette S-Konfiguration aufweisen.

Besonders bevorzugte Verbindungen der Formel I sind solche, in denen
n = 1,
R = Wasserstoff oder Alkyl mit 1 bis 4 C-Atomen,
R¹ = Wasserstoff, (C₁ bis C₃)-Alkyl, die gegebenenfalls acylierte Seitenkette von Lysin, (C₂ oder C₃)-Alkenyl, die O-(C₁ bis C₆)-alkylierte Seitenkette von Tyrosin,Benzyl, Phenethyl, 4-Amino-butyl oder Benzoylmethyl,
R² = Wasserstoff, (C₁ bis C₄)-Alkyl oder Benzyl und
X = Cyclohexyl oder Phenyl, das durch (C oder C₂)-Alkyl, (C₁ oder C₂)-Alkoxy, Hydroxy, Fluor, Chlor, Brom, Amino, (C₁ bis C4)-Alkylamino, Di-(C₁ bis C₄)-alkyl-amino, Nitro und/oder Methylendioxy, mono-oder disubstituiert oder im Falle von Methoxy trisubstituiert sein kann, bedeuten,

insbesondere solche Verbindungen der Formel I, in denen n=1, R=Wasserstoff, R¹= Methyl, R²=Wasserstoff oder Ethyl bedeuten und die chiralen C-Atome, die mit einem Stern (^{*}) gekennzeichnet sind, und C-Atom 3 die S-Konfiguration besitzen.

Die Erfindung betrifft ferner Verfahren zur Herstellung der Verbindungen der Formel I. Eine Verfahrensvariante ist dadurch gekennzeichnet, daß man eine Verbindung der Formel II 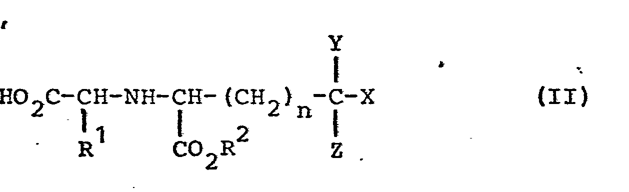 1 2 worin n, R¹, R², X, Y und Z die Bedeutungen wie in Formel I haben, mit einer Verbindung der Formel III, 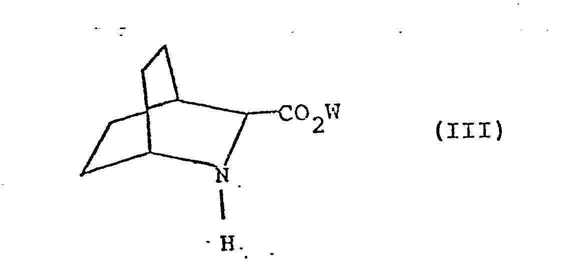 in welcher
W = Wasserstoff oder einen sauer oder hydrogenolytisch abspaltbaren Rest, insbesondere einen tert.-ButylRest oder einen Benzylrest bedeutet,

nach bekannten Amidbildungsmethoden der Peptidchemie umsetzt und gegebenenfalls anschließend durch Säurebehandlung oder Hydrierung den Rest W und gegebenenfalls durch zusätzliche Säure- oder Basenbehandlung auch den Rest R² abspaltet, wobei jeweils die freien Carbonsäuren erhalten werden.

Weitere Syntheseverfahren zur Herstellung der Verbindungen der Formel I, in der Y und Z zusammen Sauerstoff bedeuten, bestehen darin, daß man eine Verbindung der Formel IV 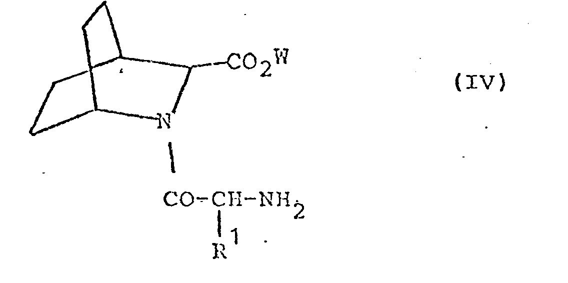 in welcher R¹ die Bedeutung wie in Formel I und W die Bedeutung wie in Formel III besitzen, mit einer Verbindung der Formel V 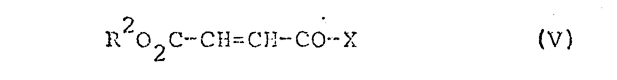 worin R² und X die Bedeutungen wie in Formel I besitzen, in bekannter Weise in einer Michael-Reaktion (Organikum, 6. Auflage, S. 492, 1967) umsetzt und gegebenenfalls den Rest W und/oder den Rest R², wie oben beschrieben, abspaltet oder daß man eine Verbindung der obengenannten Formel IV mit einer Verbindung der allgemeinen Formel VI, worin R² die Bedeutung wie in Formel I hat, und mit einer Verbindung der allgemeinen Formel VII 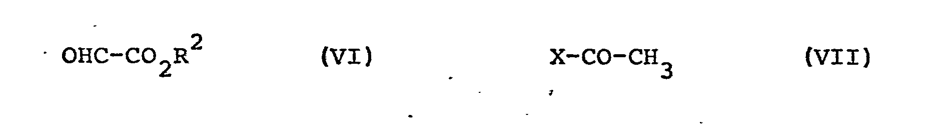 worin X die Bedeutung wie in Formel I hat, in bekannter Weise in einer Mannich-Reaktion (Bull. Soc. Chim. France 1973, S. 625) umsetzt und anschließend gegebenenfalls den Rest W und/oder den Rest R₂ wie oben beschrieben unter Bildung der freien Carboxygruppen abspaltet.

Ferner können Verbindungen der Formel I mit Y und Z jeweils = Wasserstoff auch in der Weise hergestellt werden, daß man eine Verbindung der oben genannten Formel IV gemäß der in J. Amer. Chem. Soc. 93, 2897 (1971) beschriebenen Verfahrensweise mit einer Verbindung der Formel VIII 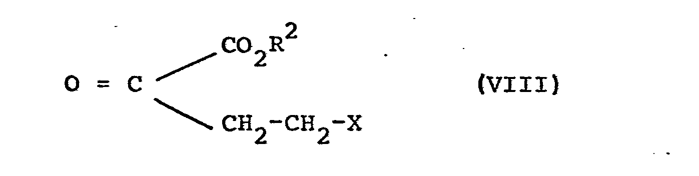 worin R² und X die Bedeutungen wie in Formel I besitzen, umsetzt, die erhaltenen Schiff-Basen,reduziert und anschließend gegebenenfalls den Rest W und/oder den Rest R², wie oben beschrieben, unter Bildung der freien Carboxygruppen abspaltet. Die Reduktion der Schiff-Basen kann elektrolytisch oder mit Reduktionsmittel wie beispielsweise Natriumborhydrid oder Natriumcyanborhydrid erfolgen.

Verbindungen der Formel I mit Y = Hydroxy und Z = Wasserstoff können beispielsweise auch durch Reduktion einer gemäß obigen Verfahrensweisenerhaltenen Verbindung _{I} mit Y und Z = zusammen Sauerstoff erhalten werden. Diese Reduktion kann mit einem Reduktionsmittel wie Natriumborhydrid und anderen komplexen Boranaten oder beispielsweise Boran-Amin-Komplexen, erfolgen.

Verbindungen der Formel I, in welcher R für Wasserstoff steht, können gegebenenfalls nach an sich bekannten Methoden in ihre Ester der Formel I, worin R (C₁ bis C₆)-Alkyl oder (C₇ bis C₉)-Aralkyl bedeutet, überführt werden.

Die Erfindung betrifft auch Verbindungen der Formel III, 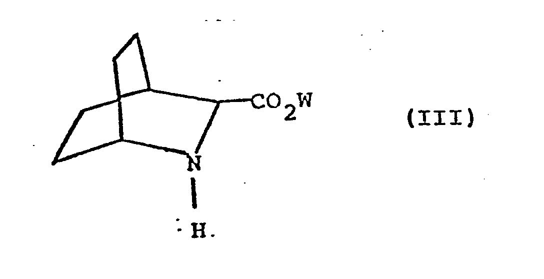 worin W = Wasserstoff oder einen sauer oder hydrogenolytisch abspaltbaren Rest wie tert.-Butyl oder Benzyl bedeutet. Diese Verbindungen dienen gemäß der Erfindung als Ausgangsstoffe bei der Synthese von Verbindungen der Formel I und können erfindungsgemäß dadurch hergestellt werden, daß man
a) eine Verbindung der Formel IX 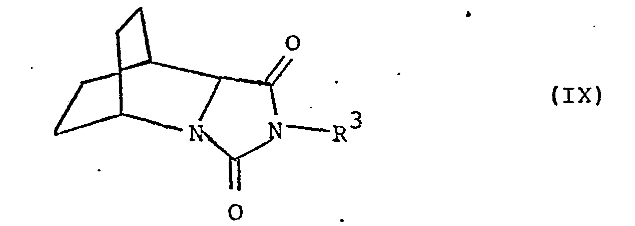 in welcher R³ einen (C₁ bis C₆)-Alkyl, Aryl-(C₁ bis C₃)-alkyl, Phenyl, 4-Methoxyphenyl- oder 4-Chlorphenylrest, insbesondere einen Methyl-, Benzyl-, Phenyl- oder 4-Chlorphenylrest darstellt, mit einem Alkali- oder Erdalkalihydroxid, insbesondere Bariumhydroxid bei 20 bis 150°C, insbesondere bei 60 bis 120°C in Wasser oder Gemischen davon mit einem organischen Lösungsmittel wie Methanol, Ethanol, Isopropanol, Tetrahydrofuran oder Dioxan verseift, und die erhaltene Aminosäure (III/W = Wasserstoff) gegebenenfalls nach üblichen Methoden der Aminosäurechemie verestert oder
b) eine Verbindung der Formel X 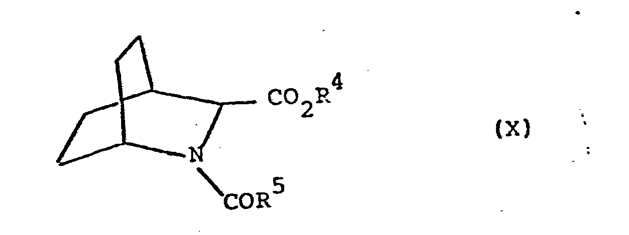 in welcher R⁴ einen (C₁ bis C₆)-Alkyl- oder Aryl-(C₁ bis C₃)-alkyl, insbesondere einen Methyl-, Ethyl-oder Benzylrest und R⁵ einen Phenyl-, Methyl-, (C₁ bis C₄)-Alkoxy- oder Aryl-(C₁ bis C₃)-alkoxy-Rest bedeuten unter den Bedingungen der Variante a) mit einem Alkali- oder Erdalkalihydroxid umsetzt und die erhaltene Aminosäure (III/W = Wasserstoff) gegebenenfalls verestert, oder
c) eine Verbindung der Formel XI 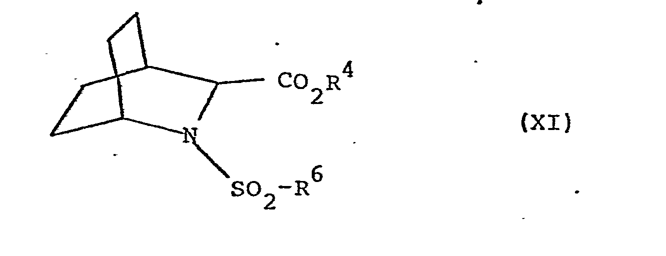 in welcher R⁴ die gleiche Bedeutung wie in Formel X besitzt und R⁶ einen Phenyl-, 4-Methylphenyl- oder 4-Chlorphenylrest darstellt, zunächst mit einem Alkali- oder Erdalkalimetall, insbesondere Natrium oder Calcium in flüssigem Ammoniak bei -60° bis - 10°, insbesondere bei -40 bis -20°C und anschließend mit einem Alkali- oder Erdalkalihydroxid, insbesondere Natrium-, Kalium oder Bariumhydroxid bei 20° bis 120°C, insbesondere 60 bis 100°C umsetzt und gegebenenfalls verestert, oder die Reihenfolge der beschriebenen Reaktionsschritte umkehrt.

Verbindungen der Formel IX sind aus Tetrahedron 27, S.3119 (1971) bekannt; Verbindungen der Formel X erhält man leicht aus den Dehydroverbindungen der Formel XII, 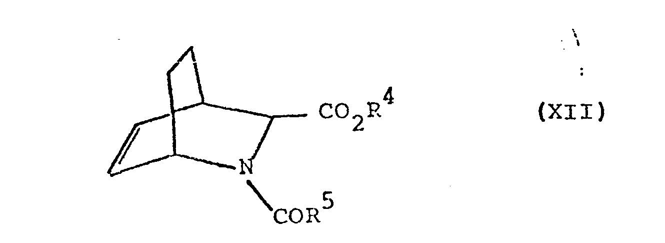 die aus J. Chem. Soc. Perkin I, S.2343 (1977) bekannt sind, durch Hydrierung in einem organischen Lösungsmittel, beispielsweise Essigester in Gegenwart eines Katalysators, beispielsweise Palladium auf Tierkohle. Verbindungen der Formel XI erhält man ebenfalls durch Hydrierung auf die eben beschriebene Weise aus den aus Chem. Ber. 98, S. 1431 (1965) bekannten Verbindungen.

Die als Ausgangsstoffe zur Herstellung der Verbindungen der Formel I verwendeten Verbindungen der Formel II mit n = 1, Y, Z = Wasserstoff, R¹ = Methyl und R² = Methyl oder Ethyl und X = Phenyl sind bekannt (EP-A-Nr. 37 231). Die Verbindungen der Formel II lassen sich nach verschiedenen Verfahrensweisen herstellen. Eine Synthesevariante geht von einem Keton der oben genannten Formel VII aus, das nach bekannten Verfahrensweisen in einer Mannich-Reaktion mit einer Verbindung der oben genannten Formel VI zusammen mit Aminosäureestern der Formel XIII 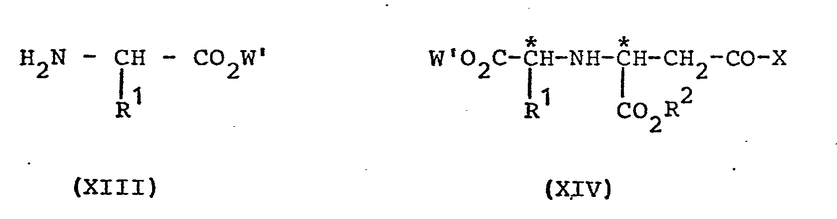 worin R¹ die oben genannte Bedeutung besitzt und W' einen hydrogenolytisch oder sauer abspaltbaren Rest, insbesondere einen Benzyl- oder einen tert.-Butyl-Rest,, bedeutet, zu einer Verbindung der Formel XIV, worin R¹₁ R², X und W' die oben genannten Bedeutungen besitzen, mit der Einschränkung, daß, wenn W' einen hydrogenolytisch abspaltbaren Rest, insbesondere Benzyl, bedeutet, R² nicht die Bedeutung von W besitzen darf, umsetzt. Spaltet man den Rest W' hydrogenolytisch mit Hilfe von beispielsweise Palladium ab, werden bei einer Wasserstoffaufnahme von 3 Moläquivalenten Verbindungen der Formel II mit Y, Z = Wasserstoff erhalten. Stoppt man die Wasserstoffaufnahme bei 1 Moläquivalent, erhält man Verbindungen der Formel II mit n = 1 und Y und Z zusammen = Sauerstoff, die man ebenfalls erhält, wenn der Rest W^{t} der Formel XIV mit Säuren, wie beispielsweise Trifluoressigsäure oder Salzsäure, in einem inerten organischen Lösungsmittel, wie beispielsweise Dioxan, abgespalten wird.

Verbindungen der Formel XIV sind auch durch Michael-Additionen einer Verbindung der oben genannten Formel V mit einer Verbindung der oben genannten Formel XIII nach bekannten Verfahrensweisen zugängliche Bevorzugt eignet sich dieses Verfahren zur Herstellung von solchen Verbindungen der Formel XI_{V}, in denen R¹ = Methyl, R2 = Ethyl und X = Aryl bedeuten.

Die Verbindungen der Formel XIV fallen als Diastereomerengemische an. Bevorzugte Diastereomeren der Formel XIV sind solche, in denen die mit einem Stern markierten chiralen C-Atome jeweils S-Konfiguration aufweisen. Diese können beispielsweise durch Kristallisation oder durch Chromatographie, z.B. an Kieselgel, abgetrennt werden. Bei der nachfolgenden Abspaltung des Restes W' bleiben die Konfigurationen der chiralen C-Atome erhalten.

Die als Ausgangssstoffe zur Herstellung der Verbindungen der Formel I verwendeten Verbindungen der oben genannten Formel IV werden aus den Verbindungen der oben genannten Formel III durch Umsetzen mit einer N-geschützten 2-Aminocarbonsäure der Formel XV 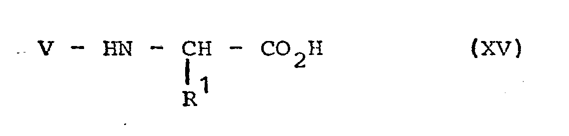 worin V eine Schutzgruppe bedeutet und R¹ die oben genannte Bedeutung besitzt, nach bekannten Verfahrensweisen erhalten. Als Schutzgruppe V, die nach beendeter Reaktion wieder abgespalten wird, kommt beispielsweise tert.-Butoxycarbonyl in Frage.

Die Umsetzung einer Verbindung der Formel II mit einer Verbindung der Formel III zur Herstellung einer Verbindung der Formel I erfolgt gemäß einer in der Peptidchemie bekannten Kondensationsreaktion, wobei als Kondensationsmittel beispielsweise Dicyclohexylcarbodiimid und 1-Hydroxy-benzotriazol zugesetzt werden. Bei der nachfolgenden sauren Abspaltung des Restes W werden als Säuren bevorzugt Trifluoressigsäure oder Chlorwasserstoff eingesetzt.

. Die gemäß oben beschriebener Verfahrensweise erhaltenen Verbindungen der Formel III fallen als Gemisch an und können beispielsweise durch Umkristallisieren oder durch Chromatographie voneinander getrennt werden.

Die Verbindungen der Formel III fallen als racemische Gemische an und können als solche in die weiteren oben beschriebenen Synthesen eingesetzt werden. Sie können aber auch nach Auftrennung der Racemate mit üblichen Methoden, beispielsweise über Salzbildung mit optisch aktiven Basen oder Säuren in die optischen Antipoden als reine Enantiomere eingesetzt werden.

Fallen die Verbindungen der Formel I als Racemate an, können auch diese nach den üblichen Methoden wie beispielsweise über Salzbildung mit optisch aktiven Basen oder Säuren in ihre Enantiomeren gespalten oder durch Chromatographie getrennt werden.

Die erfindungsgemäßen Verbindungen der Formel I, liegen, falls R = Wasserstoff ist, als innere Salze vor. Als amphotere Verbindungen können sie Salze mit Säuren oder Basen bilden. Diese Salze werden.in üblicher Weise durch Umsetzen mit einem Äquivalent Säure bzw. Base hergestellt.

Die Verbindungen der Formel I und deren Salze besitzen lang andauernde, intensive blutdrucksenkende Wirkung. Sie sind starke Hemmer des Angiotensin-Converting-Enzyms (ACE-Hemmer). Sie können zur Bekämpfung des Bluthochdrucks verschiedener Genese eingesetzt werden. Auch ihre Kombination mit anderen blutdrucksenkenden, gefäßerweiternden oder diuretisch wirksamen Verbindungen ist möglich. Typische Vertreter dieser Wirkklassen sind z.B. in Erhardt-Ruschig, Arzneimittel, 2. Auflage, Weinheim, 1972, beschrieben. Die Anwendung kann intravenös, subcutan oder peroral erfolgen.

Die Dosierung bei oraler Gabe liegt bei 1-100 mg, vorzugsweise bei 1-40 mg je Einzeldosis bei einem normalgewichtigen erwachsenen Patienten; dies entspricht etwa 15-1300 µg/kg/Tag, vorzugsweise 15-500 gg/kg/Tag. Sie kann in schweren Fällen auch erhöht werden, da toxische Eigenschaften bisher nicht beobachtet wurden. Auch eine Herabsetzung der Dosis ist möglich und vor allem dann angebracht, wenn gleichzeitig Diuretika verabreicht werden.

Die erfindungsgemäßen Verbindungen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesiumkarbonat, Kaliumphosphat, Milchzucker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren, oder weitere Hilfsstoffe in Lösung, Suspension oder Emulsion gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Äthanol, Propandiol, oder Glycerin, daneben auch Zuckerlösungen wie Glukose-oder Mannitlösungen, oder auch eine Mischung aus den verschiedenen genannten Lösungsmitteln.

Die außerordentlich starke Wirksamkeit der Verbindungen gemäß Formel I wird durch die pharmakologischen Daten der nachfolgenden Tabelle belegt:
Intraduodenale Gabe an der narkotisierten Ratte, 50 % Hemmung der durch 310 ng Angiotensin I ausgelösten Pressoreaktion 30 min. nach Applikation in der Dosis ..... = ED₅₀:

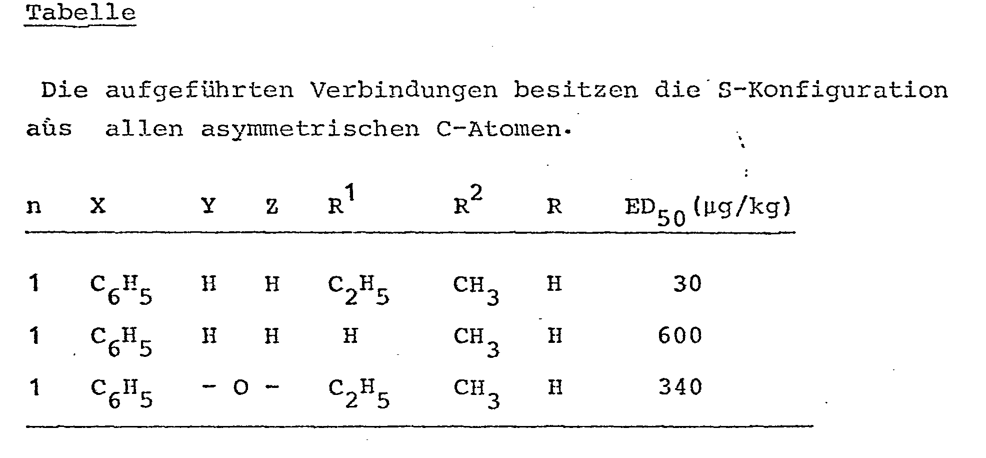

Die Symbole n, X, Y, Z, R, R¹ und R² beziehen sich auf die Verbindungen der Formel I.

Die nachfolgenden Beispiele dienen zur Erläuterung der Erfindung, ohne diese auf die stellvertretend genannten Verbindungen zu beschränken.

Die in den folgenden Beispielen angegebenen 1H-NMR-Daten wurden, wenn nicht anders angegeben, durch Messung in CDC1 ermittel und sind in δ (ppm) angegeben.

### Beispiel 1

### N-(1-S-Carbethoxy-3-phenyl-propyl)-S-alanyl-2-aza-bicyclo-[2.2.2]octan-3-S-carbonsäurebenzylester

### a) N-(p-Chlorphenyl)-2-azabicyclo[2.2.2]oct-5-en-2,3-dicarboximid

Man erhitzt eine Mischung aus 11,4 ml Cyclohexadien, 7,2 g 3-(p-Chlorphenyl)-5-methoxy-imidazolin-2,4-dion, 6 ml Trifluoressigsäure und 135 ml Toluol 18 Stunden auf 100°C. Nach dem Abkühlen und Einengen chromatographiert man an Kieselgel mit Essigester /Cyclohexan (1:2) als Laufmittel. Man erhält 1,8 g blaßgelbe Kristalle vom Schmp. 171°C.

1H-NMR-Daten:
7,7 - 7,1 (m, 4H);
6,6 - 6,1 (m, 2H);
4,7 - 4,45 (m, 1H);
4,21 (d, J= 2 Hz, 1H);
3,3 - 3,0 (m, 1H);
2,4 - 1,0 (m, 4H).

### b) N-(p-Chlorphenyl)-2-azabicyclo/2.2.27octan-2,3-dicarboximid

1,8 g N-(p-Chlorphenyl)-2-aza-bicyclo/2.2.2/oct-5-en-2,3-dicarboximid werden in 30 ml Essigester unter Zusatz von 100 mg Palladium auf Tierkohle (10 %) bei Raumtemperatur und Normaldruck hydriert. Nach Abfiltrieren und Einengen erhält man 1,8 g blaßgelbe Kristalle vom Schmp. 189°C.

1H-NMR-Daten:
7,40 - (s, 4H);
4,2 - 3,9 (m, 2H);
2,6 - 1,1 (m, 9H).

### c) 2-Azabicyclo/2.2.27octan-3-carbonsäure

1,8 g N-(p-Chlorphenyl)-2-azabicyclo[2.2.2]octan-2,3-dicarboximid werden zu 6,4 g Bariumhydroxid, Octahydrat in 30 ml Dioxan und 30 ml Wasser gegeben und 16 Stunden am Rückfluß gekocht. Nach Verdünnen mit 60 ml Wasser wird durch Zugabe von Trockeneis neutralisiert, abgesaugt und das Filtrat mit Ether extrahiert. Man engt die wässrige Phase zur Trockene ein, nimmt den Rückstand in wenig Isopropanol auf und fällt das Produkt mit Diisopropylether aus. Man erhält 0,9 g eines farblosen amorphen Pulvers.

1H-NMR-Daten (D₂0)^{:}
3,9 - 3,6 (m, 1H);
2,8 - 2,6 (m, 1H);
2,0 - 1,3 (m, 9H).
_{M}S: 155 (M⁺, 5 %); 110 (M⁺-COOH, 100 %); 82 (7_{5 %})

### d) 2-Azabicyclo[2.2.2]octan-3-carbonsäure-benzylester

Man gibt 0,9 g 2-Azabicyclo[2.2.2]octan-3-carbonsäure bei -5°C zu einer bei -20°C hergestellten Mischung von 0,9 ml Thionylchlorid in 9 ml Benzylalkohol. Nach Aufwärmen auf Raumtemperatur läßt man 16 Stunden stehen, destilliert den Benzylalkohol ab, verrührt den Rückstand dreimal mit Diisopropylether, nimmt mit 5-prozentiger Natriumcarbonatlösung auf, extrahiert dreimal mit Methylenchlorid, trocknet über Kaliumcarbonat und engt ein. Man erhält 0,5 g blaßgelbes öl.

1H-NMR-Daten:
7,1 (s, 5H);
5,0 (s, 2H);
3,9 - 3,7 (m, 1H);
2,8 - 2,6 (m, 1H);
2,0 - 1,3 (m, 9H).

### e) N-(1-S-Carbethoxy-3-phenyl-propyl)-S-alanyl-2-aza- bicyclo[2.2.2]octan-3-S-carbonsäurebenzylester (Diastereomer A1) und N₋(1-S-Carbethoxy-3-phenyl-propyl)-S-alanyl-2-aza- bicyclo[2.2.2]octan-3-R-carbonsäurebenzylester (Diastereomer B1)

0,57 g N-(1-S-Carbethoxy-3-phenyl-propyl)-S-alanin, 0,27 g N-Hydroxybenztriazol, 0,57 g 2-Azabicyclo /2.2.27octan-3-carbonsäure-benzylester sowie 0,41 g Dicyclohexylcarbodiimid werden bei Raumtemperatur in 4 ml Dimethylformamid gelöst und eine Stunde gerührt. Man verdünnt mit Essigester, filtriert den ausgefallenen Dicyclohexylharnstoff ab, wäscht mit Wasser und gesättigter Kochsalzlösung, trocknet über Natriumsulfat und engt ein. Das erhaltene Öl wird zur Trennung der Diastereomeren an Kieselgel mit Cyclohexan/Essigester (1:1) als Laufmittel chromatographiert.

Diastereomer A1: 0,27 g, R_{f}-Wert: 0,19 1H-NMR-Daten:
7,2 (s, 5H);
7,08 (s, 5H);
5,1 (s, 2H);
4,5 - 4,2 (m, 1H);
4,1 (q, J = 7 Hz, 2H);
3,9 - 1,3 (m, 16H);
1,4 - 1,1 (d+t, J = 7 Hz, 6H).

Diastereomer B1: 0,26 g, R -Wert: 0,14 1H-NMR-Daten:
7,2 (s, 5H);
7,05 (s, 5H);
5,05 (s, 2H);
4,5 - 4,2 (m, 1H);
4,15 (q, J = 7 Hz, 2H);
3,9 - 1,3 (m, 16H);
1,25 (d+t, J = 7 Hz, 6H).

### Beispiel 2

### N-(1-S-Carbethoxy-3-phenylpropyl)-S-alanyl-2-azabicyclo [2.2.2]octan-3-S-carbonsäure-Hydrochlorid

0,27 g (N-(1-S-Carbethoxy-3-phenyl-propyl)-S-alanyl-2-azabicyclo[2.2.2]octan-3-S-carbonsäurebenzylester (Diastereomer A1) werden in 20 ml Ethanol mit 50 mg Palladium auf Tierkohle bei Raumtemperatur und Normaldruck hydriert. Nach Filtrieren des Katalysators versetzt man mit 2,5 N ethanolischer Salzsäure und engt ein. Das erhaltene öl wird in wenig Methylenchlorid aufgenommen

und mit Isopropylether gefällt. Farbloses Pulver, Schmp. 185-195°C (Zers.)

1H-NMR-Daten (DMSO-d₆)

7,25 (s, 5H);

4,25 (q, 2H);

4,6 - 1,4 (m, 17H);

1,38 (d, 3H, J=7Hz);

1,25 (t, 3H, J=7Hz).

MS:
416 (M⁺, 1,5 %); 294 (19 %); 248 (11 %); 234 (100 %).

### Beispiel 3

### N-(1-S-Carbethoxy-3-phenyl-propyl)-S-alanyl-2-azabicyclo-[2.2.2]octan-3-R-carbonsäure-Hydrochlorid

0,26 g N-(1-S-Carbethoxy-3-phenyl-propyl)-S-älanyl-2-azabicyclo/2.2.2/octan-3-R-carbonsäurebenzylester (Diastereomer B1) werden in 20 ml Ethanol mit 50 mg Palladium auf Tierkohle als Katalysator bei Raumtemperatur und Normaldruck hydriert. Nach Abfiltrieren des Katalysators wird mit 2,5N ethanolischer Salzsäure versetzt eingeengt, Toluol zugesetzt und erneut eingeengt. Der kristalline Rückstand wird in Diisopropylether aufgenommen und abgesaugt, Schmp. 196-198°C

1H-NMR-Daten (DMSO-d₆):
7,22 (s, 5H);
4,2 (q, J = 7 Hz, 2H);
4,6 - 1,4 (m, 17H);
1,4 (d, J=7Hz, 3H);
1,26 (t, J=7Hz, 3H).
MS:
416 (M⁺, 0,5 %); 294 (100 %); 248 (56 %); 234 (90 %).

### Beispiel 4

### 2-Azabicyclo[2.2.2]octan-3-carbonsäure-tert.butylester Hydrochlorid

Zu einer auf -10°C gekühlten Lösung von 1,1 g 2-Azabicyclo[2.2.2]octan-3-carbonsäure in 10 ml Dioxan werden 1 ml konz. Schwefelsäure und 5 g Isobutylen gegeben. Das Reaktionsgemisch wird im Autoklaven langsam auf 20 bis 25°C erwärmt und 20 Stunden bei dieser Temperatur gerührt. Man gibt das Gemisch in eiskaltes 50 %iges wäßriges Natriumhydroxid und extrahiert mit Methylenchlorid. Die vereinigten organischen Phasen werden mit Wasser gewaschen, mit Natriumsulfat getrocknet, eingeengt, der Rückstand in Ether gelöst und gasförmiger Chlorwasserstoff eingeleitet. Das Produkt wird abgesaugt. Man erhält 0,8 g der Titelverbindung.

1H-NMR-Daten (DMSO-d₆):
3,9 - 3,6 (m, 1H);
2,9 - 2,6 (m, 1H);
2,0 - 1,3 (m, 9H);
1,3 (s, 9H).

### Beispiel 5:

### N-(1-S-Carbethoxy-3-phenyl-propyl)-S-alanyl-2-aza- bicyclo[2.2.2]octan-3-S-carbonsäure-tert.butylester (Diastereomer A5)

Analog der Verfahrensweise von Beispiel 1 erhält man aus 0,5 g 2-Azabicyclo[2.2.2]octan-3-carbonsäure-tert. butylester. Hydrochlorid, 0.57 g N-(1-S-Carbethoxy-3-phenyl-propyl)-S-alanin, 0,27 g N-Hydroxybenzotriazol sowie 0,41 g Dicyclohexylcarbodiimid unter Zusatz von 0,23 g N-Ethylmorpholin 0,28 g der Titelverbindung als farbloses öl.

1H-NMR-Daten:
7,1 (s, 5H);
4,5 - 4,1 (m, 1H);
4,1 (q, J=7Hz, 2H);
3,9 - 1,9 (m, 16E);
1,3 (s, 9H);
1,2 (d+t, J=7Hz, 6H).

### Beispiel 6

### N-(1-S-Carbethoxy-3-phenyl-propyl)-S-alanyl-2-azabicyclo [2.2.2]octan-3-S-carbonsäure Hydrochlorid

0,28 g des tert. Butylesters aus Beispiel 5 werden in 1,5 ml Trifluoressigsäure gelöst und bei O°C 3 Stunden gerührt. Die Trifluoressigsäure wird im Vakuum abgedampft, Toluol zugesetzt und nochmals eingeengt. Nach Filtration über eine kurze Kieselgelsäule mit Methylenchlorid/Ethanol (10:1) als Laufmittel wird mit ethanolischer Salzsäure sauer gestellt und eingeengt. Nach Lösen in wenig Methylenchlorid wird die Titelverbindung mit Diisopropylether ausgefällt. Man erhält 0,21 g, identisch mit der Verbindung aus Beispiel 2.

### Beispiel 7

### N-(1-S-Carboxy-3-phenyl-propyl)-S-alanyl-2-azabicyclo [2.2.2]octan-3-S-carbonsäure

Eine Lösung von 0.2 g N-(1-S-Carbethoxy-3-phenyl-propyl) -S-alanyl-2-azabicyclo/2.2.2/octan-3-S-carbonsäure Hydrochlorid in 2 ml Wasser wird mit zwei Äquivalenten Kaliumhydroxid und einem 10 %igen Überschuß 4n Kaliumhydroxidlösung versetzt. Nach 8-stündigem Rühren bei 20 bis 25°C wird die Reaktionslösung mit 2n Salzsäure auf einen pH-Wert von 4 gestellt und im Vakuum eingeengt. Man nimmt den Rückstand in Essigester auf und filtriert das abgeschiedene Salz ab. Die Essigesterlösung wird eingeengt, der Rückstand mit Diisopropylether verrieben und abgesaugt.

1H-NMR-Daten:
7,1 (s, 5H) ;
4,4 - 4,0 (m, 1H);
3,9 - 1 ,3 (m, 16H);
1,2 (d, 3H).

### Beispiel 8

### N-(1-S-Carbethoxy-3-phenyl-3-oxo-propyl)-S-alanyl-2-azabicyclo[2.2.2]octan-3-S-carbonsäure-tert.butylester

0,24 g 2-Azabicyclo/2.2.2/octan-3-carbonsäure-tert. butylester Hydrochlorid werden zusammen mit 0,14 g 1-Hydroxybenzotriazol, 0,29 g N-(1-S-Carbethoxy-3-phenyl-3-oxo-propyl)-S-alanin, 0,22 g Dicyclohexylcarbodiimid und 0,12 g N-Ethylmorpholin in 3 ml DMF gelöst und 3 Stunden bei 20°C gerührt. Nach Verdünnen mit Essigester wird filtriert, zweimal mit Wasser gewaschen, getrocknet und eingeengt. Der Rückstand wird an Kieselgel mit Cyclohexan/Essigester (1:1) als Laufmittel chromatographiert; man erhält 0,16 g der Titelverbindung.

1H-NMR-Daten:
8,2 - 7,1 (m, 5H);
4,7 - 4,1 (m, 1H);
4,1 (q, J=7Hz, 2H);
3,9 - 1,3 (m, 14H);
1 ,3 (^{s}, 9H);
1,2 (d+t, 6H).

### Beispiel 9

### N-(1-S-Carbethoxy-3-phenvl-3-oxo-propyl)-S-alanyl-2-azabicyclo[2.2.2]octan-3-S- carbonsäure.Hydrochlorid

0,16 g der Verbindung aus Beispiel 8 werden analog dem Verfahren von Beispiel 6 mit 2 ml Trifluoressigsäure zu 0,12 g der Titelverbindung umgesetzt.

1H-NMR-Daten (DMSO-d₆):
8,1 - 7,2 (m, 5H);
4,7 - 4,1 (m, 1H)
4,1 (q, J=7Hz, 2H);
3,9 - 3,1 (m, 14H);
1,2 (d+t, 6H).

### Beispiel 10

### N-(1-S-Carboxy-3-phenyl-3-oxo-propyl)-S-alanyl-2-aza- bicyclo[2.2.2]octan-3-S-carbonsäure

0,19 g der Verbindung aus Beispiel 9 werden mit 2,2 Äquivalenten Kaliumhydroxid analog der in Beispiel 7 beschriebenen Verfahrensweise umgesetzt.

1H-NMR-Daten:
8,1 - 7,2 (m, 5H);
4,7 - 4,1 (m, 1H);
3,9 - 3,1 (m, 14H);
1,2 (d, J=7_{H}z, 3H).

### Beispiel 11

### S-Alanyl-2-azabicyclo[2.2.2]octan-3-S-carbonsäure- tert.butylester

### a) N-Methylsulfonylethyloxycarbonyl (MSC)-S-alanyl-2-azabicyclo[2.2.2]octan-3-S-carbonsäure-tert.butylester

Zu einer Lösung von 10 g MSC-Ala-OH in 50 ml Dimethylformamid werden 6,7 g 1-Hydroxybenzotriazol und 14,0 g 2-Azabicyclo[2.2.2]octan-3-carbonsäure-tert.butylester gegeben. Der pH-Wert wird mit N-Ethylmorpholin auf 8,0 eingestellt. Das Gemisch wird im Eisbad gekühlt und mit 10,5 g Dicyclohexylcarbodiimid versetzt.

Man rührt 15 Stunden bei 20 - 25°C. Der ausgefallene Harnstoff wird abgesaugt, das Filtrat im Vakuum eingeengt, und in Essigester aufgenommen. Die organische - Phase wird mit nacheinander Kaliumhydrogensulfat-, Kaliumhydrogencarbonat- und Natriumchloridlösung gewaschen, getrocknet und eingedampft. Der Rückstand wird an Kieselgel mit Essigester/Cyclohexan 1:1 chromatographiert. Ausbeute: 10 g

1H-NMR-Daten:
4,8 - 3,8 (m, 2H);
3,8 - 3,1 (m, 5H);
3,0 (s, 3H);
2,9 - 1,2 (m, 9H);
1,4 (s, 9H);
1,2 (d, J=7Hz, 3H).

### b) S-Alanyl-2-azabicyclo[2.2.2]octan-3-S-carbonsäure- tert.butylester

2,0 g der Verbindung aus Beispiel 10 a werden in 15 ml Methanol und 1,5 ml Wasser gelöst. Es wird mit 2n Natronlauge auf pH 13 gebracht und 2 Stunden bei Raumtemperatur gerührt. Danach wird mit 2n Salzsäure neutralisiert, das Methanol im Vakuum abgedampft,-die wäßrige Phase mit Essigester extrahiert, die Essigesterlösung mit Wasser gewaschen, getrocknet und eingeengt. Der Rückstand wird über Kieselgel mit Essigester als Elutionsmittel filtriert.

Ausbeute: 0,8 g

1H-NMR-Daten:
4,7 - 4,2 (m, 1H);
3,9 - 3,3 (m, 2H);
2,9 - 1 ,2 (m, 9H);
1 ,4 (s, 9H);
1,2 (d, J=7_{H}z, 3H).

### Beispiel 12

### N-(1-S-Carbethoxy-3-oxo-3-phenyl-propyl)-S-alanyl₋2-azabicyclo[2.2.2]octan-3-S-carbonsäure-tert.butylester

5m Mol der Verbindung aus Beispiel 11bwerden zusammen mit 5 m Mol 3-Benzoyl-acrylsäureethylester und 5 Tropfen Triethylamin in 50 ml wasserfreiem Ethanol gelöst und das Gemisch 24 Stunden bei 20 bis 25°C gerührt. Es wird zur Trockene eingedampft und der Rückstand in Essigester aufgenommen. Nun wird mit Wasser gewaschen, getrocknet und eingedampft.

Das Diastereomerengemisch wird an Kieselgel mit Essigester/Cyclohexan als Elutionsmittel chromatographiert. Die ¹H-NMR-Daten stimmen mit den Daten der Verbindung aus Beispiel 8 überein.

### Beispiel 13

### N-(1-S-Carbethoxv-3-phenyl-propyl)-S-alanyl-2-azabicyclo-[2.2.2]octan-3-S-carbonsäure-tert.butylester

5 mmol S-Alanyl-2-azabicyclo[2.2.2]octan-3-S-carbonsäure- tert.butylester werden in 15 ml wasserfreiem Ethanol gelöst. Man stellt die Lösung mit ethanolischem Kaliumhydroxid auf pH 7,0 und gibt 0,7 g pulverisiertes Molekularsieb (4 Å) und anschließend 5 m Mol 2-Keto-4-phenyl-buttersäureethylester dazu. Es wird eine Lösung von 0,6 g Natriumcyanborhydrid in 6 ml wasserfreiem Ethanol langsam zugetropft. Nach einer Reaktionszeit von 20 Stunden bei 20 bis 25°C wird die Lösung filtriert und das Lösungsmittel abdestilliert.'Der Rückstand wird in Essigester/Wasser aufgenommen. Nach dem Eindampfen der Essigesterphasen wird der Rückstand an Kieselgel mit Essigester/Cyclohexan 1:4 chromatographiert.

Die ¹H-NMR-Daten stimmen mit den Daten der Verbindung aus Beispiel 5 überein.

### Beispiel 14

### N-(1-S-Carbethoxy-3-phenyl-3-oxo-propyl)-S-alanyl-2-azabicyclo/2.2.2/octan-3-S-carbonsäure-tert.butylester

10 mmol Acetophenon, 10 mmol Glyoxylsäureethylester und 10 mmol S-Alanyl-2-azabicyclo/2.2.2/octan-3-S-carbonsäure tert.-butylester werden in 30 ml Eisessig 36 Stunden auf 45°C erhitzt. Nach dem Einengen im Vakuum wird mit Natriumbicarbonatlösung neutral gestellt und mit Essigester extrahiert. Die Essigesterphase wird eingeengt und an Kieselgel mit Essigester/Cyclohexan 1:1 als Elütionsmittel chromatographiert. Die NMR-Daten stimmen mit den Daten der Verbindung aus Beispiel 8 überein.

### Beispiel 15:

### N-(1-S-Carbethoxy-3-R,S-hydroxy-3-phenyl-propyl)-S-alanyl-2-azabicyclo/2.2.27octan-3-S-carbonsäure

0,5 g N-(1-S-Carbethoxy-3-oxo-3-phenyl-propyl)-S-alanyl-2-azabicyclo[2.2.2]octan-3-S-carbonsäure werden in 5 ml wäßrigem Ethanol gelöst und 0,1 g Natriumborhydrid zugegeben. Es wird 14 Stunden bei Raumtemperatur gerührt. Danach wird mit Essigester versetzt, die Essigesterlösung mit Wasser gewaschen, getrocknet und eingedampft. Das Rohprodukt wird über Kieselgel mit Essigester/Methanol 9:1 als Elutionsmittel filtriert. Ausbeute: 0,3 g.

1H-NMR-Daten:
7,3 - 6,9 (m, 5H);
5,4 (t, 1H) ;
4,7 - 4,2 (m, 1H);
3,9 - 1 ,3 (m, 14H);
1,3 (d+t, 16H) .

### Beispiel 16

### 2-Aza-bicyclo[2.2.2]octan-3-carbonsäure

### a) 2(p-Toluolsulfonyl)-2-azabicyclo[2.2.2]oct-5-en- carbonsäure-butylester

28 g N-[Butyloxycarbonylmethylen]-p-toluolsulfonamid und 10 g Cyclohexadien werden in 50 ml Toluol 12,5 Stunden auf 80°C erhitzt, Nach Abkühlen wird eingeengt.

1H-NMR-Daten :
7,4 - 6,9 (m, 4H);
5,6 - 4,2 (m, 4H);
4,1 (t , J=7Hz , 2H) ;
2,3 (s, 3H)
2,4 - 1,2 (m, 7H);
1 ,0 (t , 3H) .

### b) 15 g der Verbindung aus Beispiel 16 a werden in 200 ml Essigester mit 0,5 g Palladium auf Kohle (10 %) bei Raumtemperatur und Normaldruck hydriert. Nach Abfiltrieren des Katalysators wird eingeengt.

1H-NMR-Daten :
. 7,4 - 6 ,9 (m, 4H);
5 ,3 - 4 ,2 (m, 2H);
4,1 (t, 3H);
3,9 - 3,3 (m, 1H);
2,3 (s, 3H);
2,4 - 1,2 (m, 11H) ;
1 ,0 (t, 3H).

### c) 2-Azabicyclo[2.2.2]octan-3-carbonsäure

4 g der Verbindung aus Beispiel 16 b werden mit 1,2 Äquivalenten KOH in 20 ml Wasser 4 Stunden auf 60°C erwärmt. Nach Neutralisieren mit 1N HCl wird eingeengt. Zum Rückstand werden 50 ml flüssiger Ammoniak gegeben und soviel Natrium, bis die blaue Farbe bestehen bleibt. Nach 3 Stunden bei -30°C wird wasserfreies Natriumacetat zugesetzt, bis die Farbe verschwindet. Nach Abdampfen des Ammoniaks wird das Rohprodukt auf einer sauren Ionenaustauschersäule gereinigt. Die analytischen Daten stimmen mit der Verbindung aus Beispiel 1 c überein.

### Beispiel 17

### 2-Aza-bicyclo/2.2.2/octan-3-carbonsäure

### a) 2-Benzyloxycarbonyl-2-azabicyclo/2.2.2/oct-5-en-3-carbonsäure-methylester

4 ml Bortrifluorid-Etherat werden unter Rühren zu einer Mischung aus N-Benzyloxycarbonyl-2-methoxy-glycin- methylester (20,2 g) und 60 ml Toluol gegeben. Bei 80°C werden 8 ml Cyclohexadien in 16 ml Toluol zugetropft. Nach 1,5 Stunden bei 80°C wird abgekühlt und auf 100 ml einer wässrigen Natriumbicarbonatlösung gegossen, über Magnesiumsulfat getrocknet, eingeengt und an Kieselgel mit Petrolether/Ether (2:1) als Laufmittel chromatographiert. Man erhält 10,6 g der Titelverbindung als exo/endo-Gemisch.

### b) 2-Azabicyclo[2.2.2]octan-3-carbonsäuremethylester

5,4 g der Verbindung aus Beispiel 17 a werden in 200 ml Essigester mit 0,5 g Palladium auf Tierkohle als Katalysator bei Raumtemperatur und Normaldruck hydriert. Nach Aufnahme von 2 Mol Wasserstoff wird der Katalysator abfiltriert und das Filtrat eingeengt. Ausbeute: 3,4 g.

1H-NMR-Daten:
3,9 - (s, 3H);
3,8 - 3,5 (m, 1H);
2 ,9 - 2,6 (m, 1H);
2,0 - 1 ,3 (m, 9H) .

Nach den in den Beispielen 1e sowie 2 beschriebenen Verfahren wurden weiterhin unter Verwendung der entsprechende Ausgangsstoffe hergestellt:

### Beispiel 18

### N-(1-S-Carbethoxy-3-cyclohexylproply)-S-alanyl-2-aza- bicyclo[2.2.2]-octan-3-S-carbonsäure-Hydrochlorid

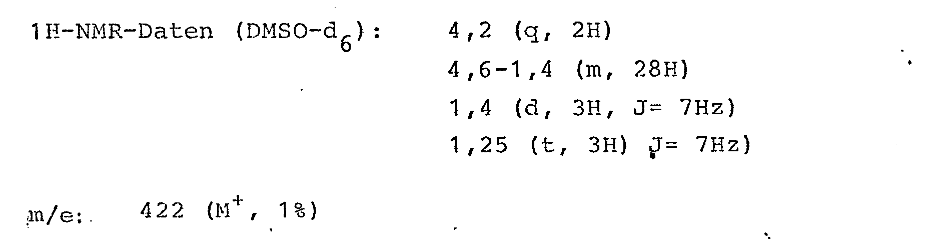

### Beispiel 19

### N- (1-S-Carbethoxy3-cyclopentylpropyl)-S-alanyl-2-aza- bicyclo[2.2.2]octan-3-S-carbonsäure-Hydrochlorid

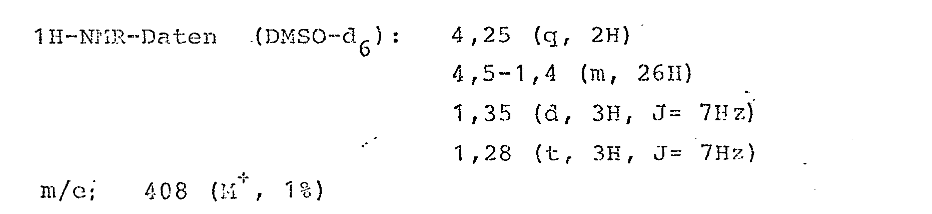

### Beispiel 20

### N-(1-S-Carbethoxy-4,4-dimeth^{y}lpentyl)-S-alanyl-2-azabicyclo[2.2.2]octan-3-S-carbonsäure-Hydrochlorid

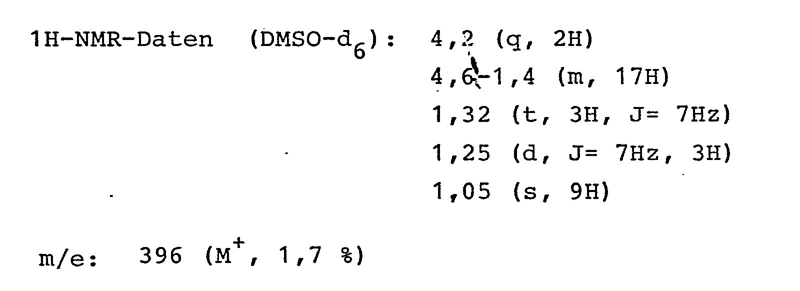

### Beispiel 21

### N-(1-S-_{C}arboethoxy-3(2,6-dimethylphenyl)-propyl)-S-alanyl-2-azabicyclo[2.2.2]-octan-3-S-carbonsäure-Hydrochlorid

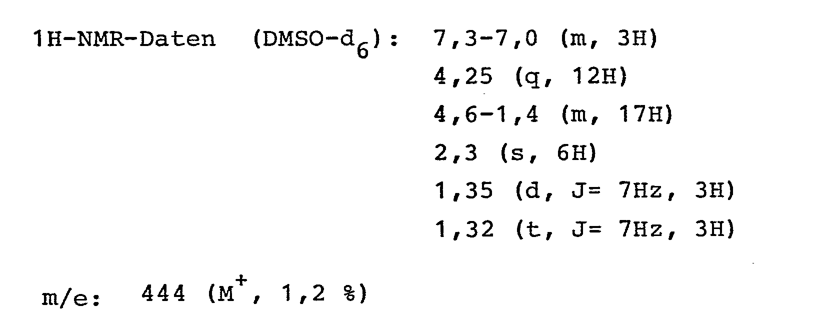

### Beispiel 22

### N-(1-S-Carboethoxy-3-(4-methoxyphenyl)-propyl)-S-alanyl-2-azabicyclo/2.2.27-octan-3-S-carbonsäure-Hydrochlorid

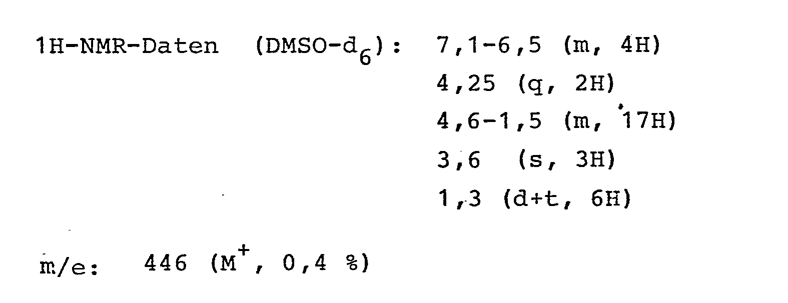

### Beispiel 23

### N-(1-S-Carbethoxy-3-(3,4-dimethoxyphenyl)-propyl)-S-alanyl-2-azabicyclo/2.2.2/-octan-3-S-carbonsäure-Hydrochlorid

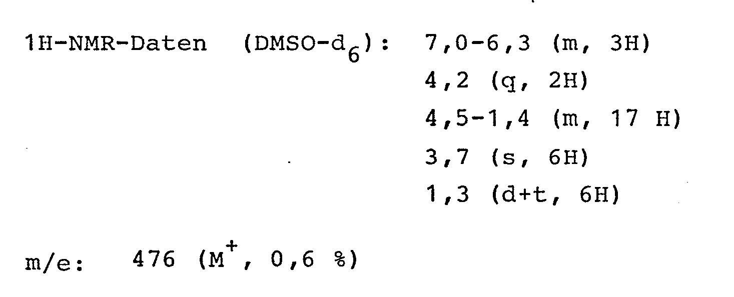

### Beispiel 24

### N-(1-S-Carbethoxy-3-(4-fluorphenyl)-propyl)-S-alanyl-2-azabicyclo/2.2.27-octan-3-S-carbonsäure-Hydrochlorid

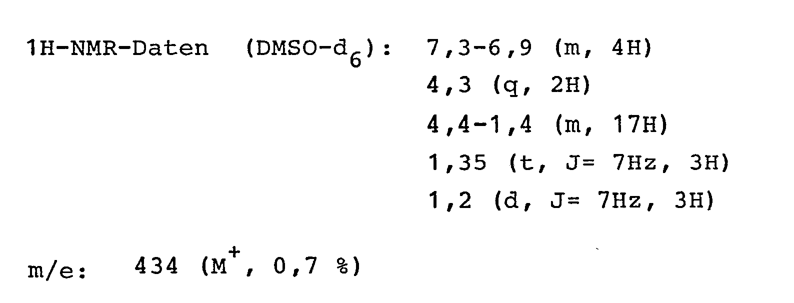

### Beispiel 25

### N-(1-S-Carbethoxy-3-(2,6-dichlorphenyl)-propyl)-S-alanyl-2-azabicyclo[2.2.2]-octan-3-S-carbonsäure-Hydrochlorid

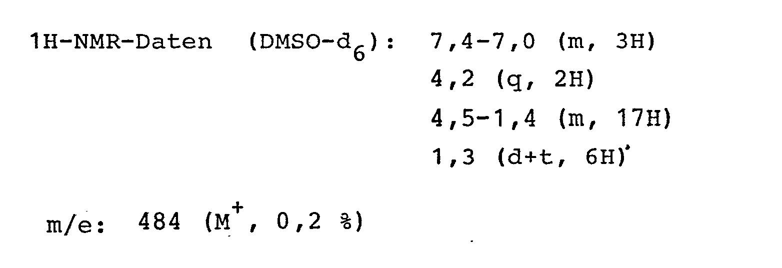

### Beispiel 26

### N-(1-S-Carbethoxy-3-(3,4-methylendioxyphenyl)-propyl)-S-alanyl-2-azabicyclo[2.2.2]-octan-3-S-carbonsäure-Hydrochlorid

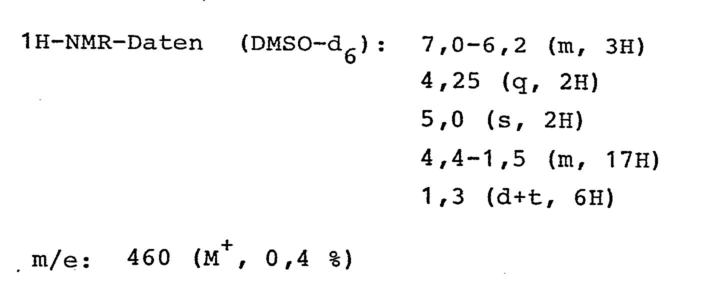

### Beispiel 27

### N-(1-S-Carbethoxy-3-phenyl-propyl)-O-ethyl-S-tyrosyl-2-azabicyclo[2.2.2]-octan-3-S-carbonsäure-Hydrochlorid

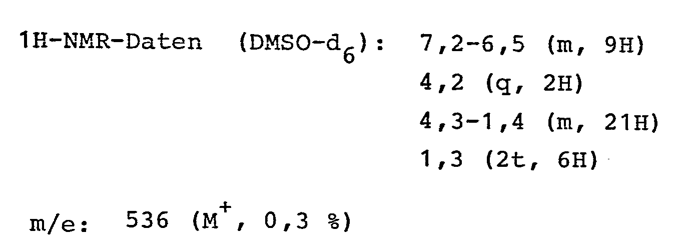

### Beispiel 28

### N-(1-S-Carbethoxy-3-phenyl-propyl)-O-methyl-S-tyrosyl-2-azabicyclo[2.2.2]-octan-3-S-carbonsäure-Hydrochlorid

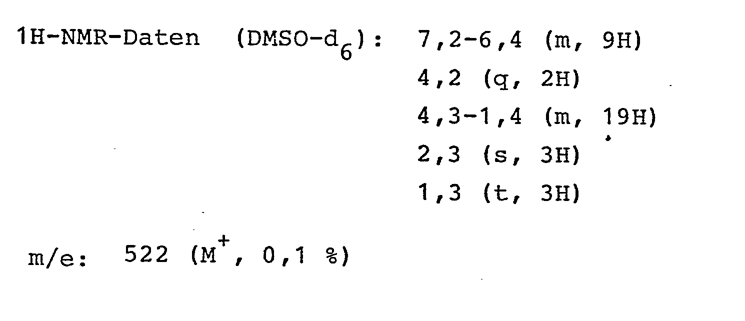

### Beispiel 29

### N-(1-S-Carboethoxy-3-cyclohexyl-propyl)-O-ethyl-S-tyrosyl-2-azabicyclo[2.2.2]octan-3-S-carbonsäure-Hydrochlorid

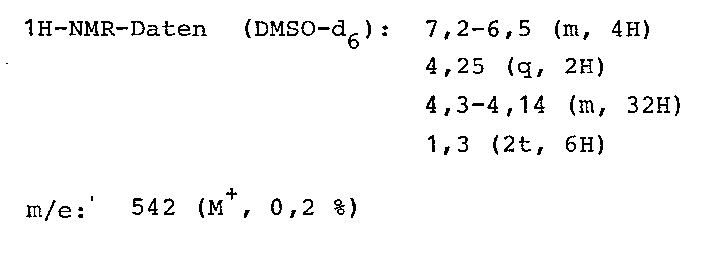

### Beispiel 30

### N-(1-S-Carbethoxy-3-cyclohexyl-propyl)-S-lysyl-2-azabicyclo[2.2.2]-octan-3-S-carbonsäure Hydrochlorid

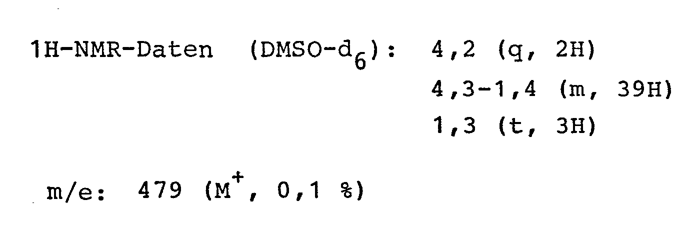

### Beispiel 31

### N-(1-S-Carboxy-3-cyclohexylpropyl)-S-lysyl-2-azabicyclo[2.2.2]-octan-3-S-carbonsäure

m/e: 451 (M⁺, 0,1 %)

## Patentansprüche

1. Verbindungen der Formel I, 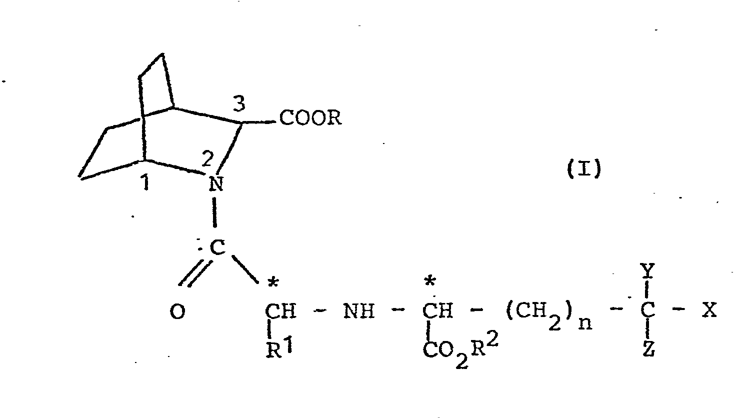 in welcher
n . = O oder 1 ,
R = Wasserstoff, (C₁ bis C₆)-Alkyl oder Aralkyl mit 7 bis 9 C-Atomen,VV,
R¹ = Wasserstoff oder (C bis C₆)-Alkyl, das gegebenenfalls durch Amin^{o}, (C₁ bis C₄)-Acylamino oder Benzoylamino substituiert sein kann, (C₂ bis C₆)-Alkenyl, (C₅ bis C₉)-Cycloalkyl, (C₅ bis C₉)-Cycloalkenyl, (C₅ bis C₇)-Cycloalkyl-(C₁ bis C₄) - alkyl, (C₆ bis C₁₀)-Aryl oder teilhydriertes (C₆ bis C₁₀)-Aryl, das jeweils durch (C₁ bis C₄)-Alkyl, (C₁ oder C₂)-Alkoxy oder Halogen substituiert sein kann, (C₆ bis C₁₀)-Aryl-(C₁ bis C₄)-alkyl oder (C₇ bis C₁₁) -Aroyl-(C₁ bis C₄)-alkyl die beide wie vorstehend definiert, im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine gegebenenfalls geschützte Seitenkette einer natürlich vorkommenden α-Auinosäure,
R² = Wasserstoff, (C₁ bis C₆)-Alkyl , (^{C}₂ bis ^{C}₆)-Alkenyl oder C₆ bis C₁₀) -Aryl- (C₁ bis C₄) -alkyl
Y = Wasserstoff oder Hydroxy,
Z = Wasserstoff oder
Y und Z = zusammen Sauerstoff und
X = (C₁ bis C₆) -Alkyl , (C₂ bis C₆) -Alkenyl, (C₅ bis Cg)-Cycloalkyl, (C₆ bis C₁₀)-Aryl, das durch (C₁ bis C₄)-Alkyl, (C₁ bis C₄)-Alkoxy, Hydroxy, Halogen, Nitro, Amino, (C₁ bis C₄)-Alkylamino , Di-(C₁ bis C₄)alkyl-amino und/oder Methylendioxy mono-, di-oder trisubstituiert sein kann, oder 3-Indolyl
bedeuten,
sowie deren physiologisch unbedenkliche Salze.

2. Verbindungen der Formel I gemäß Anspruch 1, dadurch gekennzeichnet, daß das C-Atom in Position 3 des bicyclischen Ringsystems sowie die mit einem Stern markierten C-Atome der Seitenkette jeweils S-Konfiguration aufweisen.

3. Verbindungen der Formel I gemäß einem der Ansprüche 1 oder 2, dadurch gekennzeichnet, daß
n = 1
R = Wasserstoff oder (C₁ bis C₄)-Alkyl,
R¹ = Wasserstoff, (C₁ bis C₃)-Alkyl, die gegebenenfalls acylierte Seitenkette von Lysin, (C₂ oder C₃)-Alkenyl, Benzyl, die O-(C₁ bis C₆)-alkylierte Seitenkette von Tyrosin, Phenethyl, 4-Amino-butyl oder Benzoylmethyl
R² = Wasserstoff, (C₁ bis C₄)-Alkyl oder Benzyl,
X = Cyclohexyl oder Phenyl, das durch (C₁ oder C₂)-Alkyl, (C₁ oder C₂)-Alkoxy, Hydroxy, Fluor, Chlor, Brom, Amino, (C₁ bis C₄)-Alkylamino, Di-(C₁ bis C₄)alkyl-amino, Nitro und/ oder Methylendioxy mono- oder disubstituiert oder, im Falle von Methoxy, trisubstituiert sein kann, bedeuten.

4. Verfahren zur Herstellung der Verbindungen der Formel I gemäß einem der Ansprüche 1 bis 3, dadurch gekennzeichnet,
a) daß man eine Verbindung der Formel II, 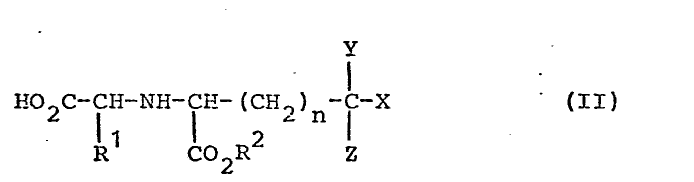 worin n, R¹ , R², X, Y und Z die Bedeutung wie in Formel I haben mit einer Verbindung der Formel III, 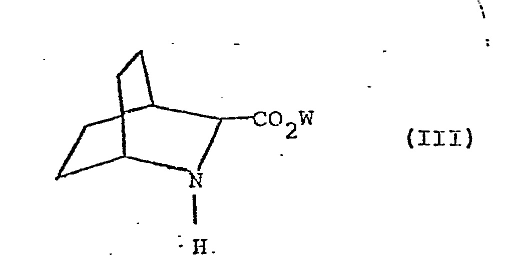 in welcher
W = Wasserstoff oder einen sauer oder hydrogenolytisch abspaltbaren Rest bedeutet, umsetzt und anschließend gegebenenfalls W und/oder R² unter Bildung der freien Carboxygruppen abspaltet, oder
daß man zur Herstellung der Verbindungen der Formel I; in der Y und Z zusammen Sauerstoff bedeuten,
b₁) eine Verbindung der Formel IV 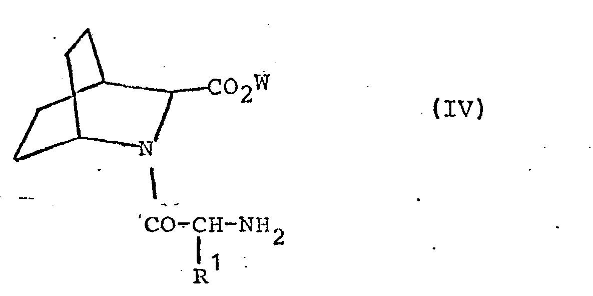 in welcher R¹ die Bedeutung wie in Formel I und W die Bedeutung wie in Formel III besitzen, mit einer Verbindung der Formel V, 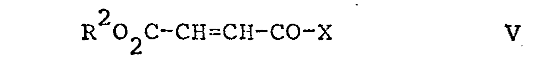 worin R² und X die Bedeutungen wie in Formel I haben, umsetzt und anschließend gegebenenfalls W undjoder R² unter Bildung der freien Carboxygruppen abspaltet, oder
b2 eine Verbindung der unter b₁) genannten Formel IV mit einer Verbindung der allgemeinen Formel VI, worin R² die Bedeutung wie in Formel I hat, und mit einer Verbindung der allgemeinen Formel VII, 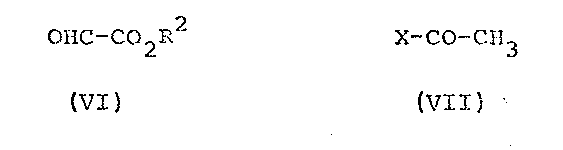 worin X die Bedeutung wie in Formel I hat, umsetzt und anschließend gegebenenfalls W und/oder unter Bildung der freien Carboxygruppen abspaltet, oder
c, daß man zur Herstellung von Verbindungen der Formel I, in der Y und Z jeweils Wasserstoff bedeuten, eine Verbindung der unter b₁) genannten Formel IV mit einer Verbindung der Formel VIII, 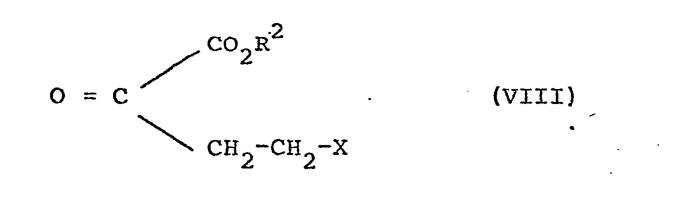 worin R² und X die Bedeutungen wie in Formel I besitzen, umsetzt, die erhaltenen Schiff-Basen reduziert und anschließend gegebenenfalls W und/oder R² unter Bildung der freien Carboxygruppen abspaltet oder
d) daß man zur Herstellung von Verbindungen der Formel I, in der Y = Hydroxy und Z = Wasserstoff bedeuten, eine Verbindung der Formel I, in der Y und Z zusammen Sauerstoff bedeuten, mit einem komplexen Boranat oder Boran-Amin-Komplex reduziert.
die nach a) bis d) erhaltenen Verbindungen der Formel I, in welcher R für Wasserstoff steht, gegebenenfalls in Ester der Formel I, worin R (C₁ bis C6)-Alkyl oder (C₇ bis C₉)-Aralkyl bedeutet, überführt und Verbindungen der Formel I gegebenenfalls in ihre physiologisch unbedenklichen Salze überführt.

5. Verwendung einer Verbindung gemäß einem der Ansprüche 1 bis 3 als Heilmittel.

6. Verbindung gemäß einem der Ansprüche 1 bis 3 zur Verwendung als Heimittel.

7. Mittel, enthaltend eine Verbindung gemäß einem der Ansprüche 1 bis 3.

8. Verbindung der Formel III, in welcher W die in Anspruch 4 definierte Bedeutung hat.

9. Verfahren zur Herstellung von Verbindungen der Formel III gemäß Anspruch 8, dadurch gekennzeichnet, daß man
a) Verbindungen der Formel IX, 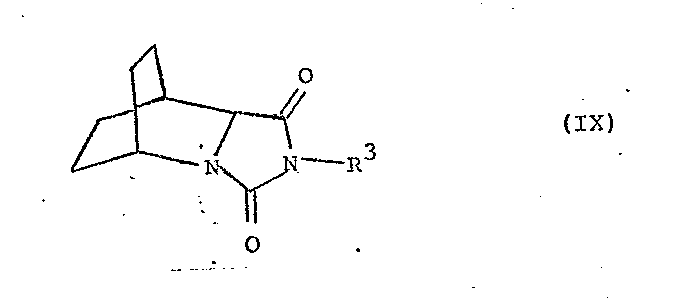 in welcher R³ einen (C₁-C₆)-Alkyl , Aryl-(C₁-C₃)-alkyl, Phenyl, 4-Methoxyphenyl- oder 4-Chlorphenylrest darstellt, mit einem Alkali- oder Erdalkalihydroxid, in Wasser oder Gemischen davon mit einem organischen Lösungsmittel verseift, und die erhaltene Aminosäure (III/W=Wasserstoff) gegebenenfalls nach üblichen Methoden der Aminosäurechemie verester oder
b) eine Verbindung der Formel X 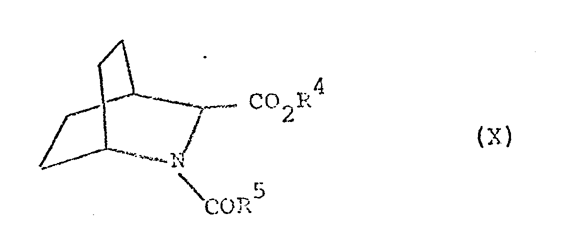 in welcher R⁴ einen (C₁ bis C₆) - oder Aryl-(C₁-C₃)-alkyl-, und R⁵ einen Phenyl-, Methyl-, (C₁ bis C₄)-Alkoxy- oder Aryl-(C₁ bis C₃)-alkoxy-Rest bedeuten unter den Bedingungen der Variante a) mit einem Alkali- oder Erdalkalihydroxid umsetzt und die erhaltene- Aminosäure (III, W = Wasserstoff) gegebenenfalls verestert, oder
c) eine Verbindung der Formel XI 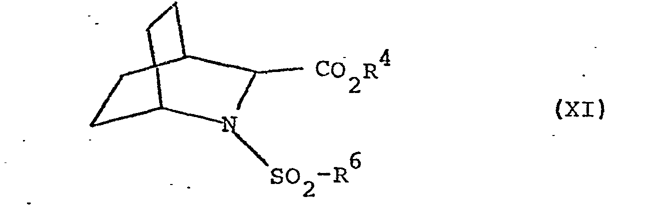 in welcher R⁴ die gleiche Bedeutung wie in FOrmel X besitzt und R⁶ einen Phenyl-, 4-Methylphenyl- oder 4-Chlorphenylrest darstellt zunächst mit einem Alkali- oder Erdalkalimetall, in flüssigem Ammoniak bei -60° bis -10°C, und anschließend mit einem Alkali-oder Erdalkalihydroxid bei 20° bis 120°C, insbesondere 60 bis 100°C, umsetzt und gegebenenfalls verestert, oder die Reihenfolge der beschriebenen Reaktionsschritte umkehrt.

10. Verwendung einer Verbindung gemäß einem der Ansprüche 1 bis 3, in Kombination mit einem Diuretikum als Heilmittel.

11. Mittel, enthaltend eine Verbindung gemäß einem der Ansprüche 1 bis 3 in Kombination mit einem Diuretikum.

PATENTANSPRÜCHE für den Vertragsstaat Österreich:

1. Verfahren zur Herstellung der Verbindungen der Formel I, 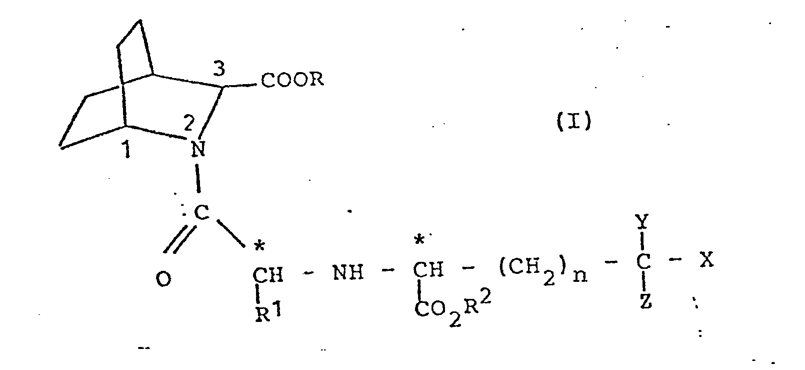 in der
n . = O oder 1,
R = Wasserstoff, (C₁ bis C₆)-Alkyl oder Aralkyl mit 7 bis 9 C-Atomen,
R¹ = Wasserstoff oder (C₁ bis C₆)-Alkyl , das gegebenenfalls durch Amin^{o}, (C₁ bis C₄)-Acylamino oder _{B}enzoylamino substituiert sein kann, (C₂ bis C₆)-Alkenyl, (C₅ bis C₉)-Cycloalkyl, (C₅ bis C₉)-Cycloalkenyl, (C₅ bis C₇) -Cycloalkyl-(C₇ bis C₄) - alkyl, (C₆ bis C₁₀)-Aryl oder teilhydriertes (C₆ bis C₁₀)-Aryl, das jeweils durch (C₁ bis C₄)-Alkyl, (C₁ oder C₂)-Alkoxy oder Halogen substituiert sein kann, (C₆ bis C₁₀)-Aryl-(C₁ bis C₄)-alkyl oder (C₇ bis C₁₁) -Aroyl-(C₁ bis C₄)-alkyl die beide wie vorstehend definiert, im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine gegebenenfalls geschützte Seitenkette einer natürlich vorkommenden α-Aminosäure,
R² = Wasserstoff, (C₁ bis C₆)-Alkyl, (C₂ bis C₆)-Alkenyl oder C₆ bis C₁₀)-Aryl-(C₁ bis C₄)-alkyl.
Y = Wasserstoff oder Hydroxy,
Z = Wasserstoff oder
Y und Z = zusammen Sauerstoff und
X = (C₁ bis C₆)-Alkyl , (C₂ bis C₆)-Alkenyl , (C₅ bis C₉)-Cycloalkyl, (C₆ bis C₁₀)-Aryl, das durch (C₁ bis C₄) - Alkyl, (C₁ bis C₄) -Alkoxy , Hydroxy , Halogen , Nitro, Amino, (C₁ bis C₄)-Alkylamino, Di-(C bis C₄)alkyl-amino und/oder Methylendioxy mono-, di-oder trisubstituiert sein kann, oder 3-Indolyl
bedeuten,
sowie deren physiologisch unbedenkliche Salze, dadurch gekennzeichnet,
a) daß man eine Verbindung der Formel II, 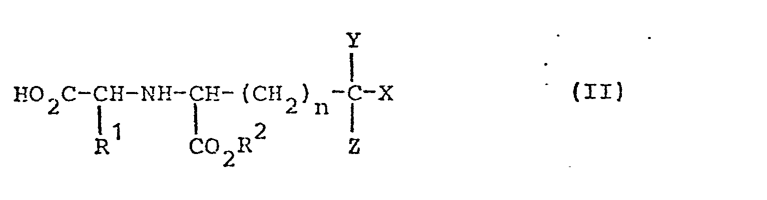 worin n, R¹, R², X , Y und Z die Bedeutung wie in Formel I haben mit einer Verbindung der Formel III, 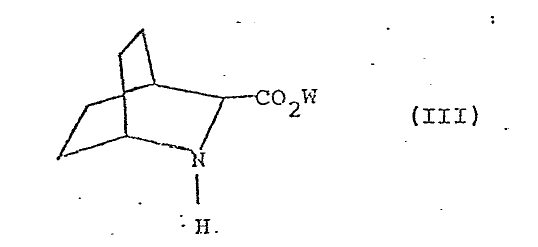 in welcher
W = Wasserstoff oder einen sauer oder hydrogonolytisch abspaltbaron Rest bedeutet, umsetzt und anschließend gegebenenfalls _{W} und/oder _{R}² unter Bildung der freien Carboxygruppen abspaltet, oder
b) daß man zur Herstellung der Verbindungen der Formel I; in der Y und Z zusammen Sauerstoff bedeuten,
b₁) eine Verbindung der Formel IV 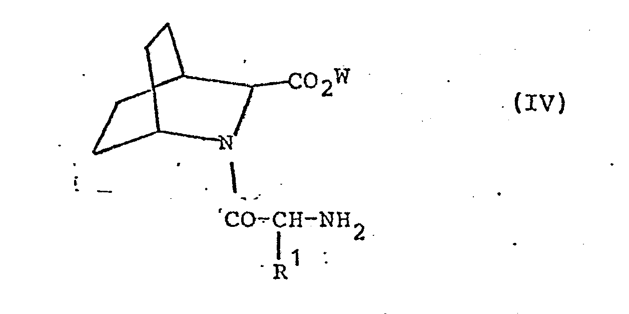 in welcher R¹ die Bedeutung wie in Formel I und W die Bedeutung wie in Formel III besitzen, mit einer Verbindung der Formel V, 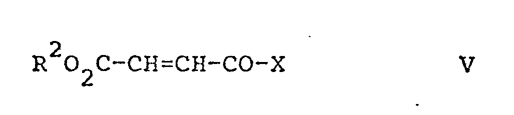 worin R² und X die Bedeutungen wie in Formel I haben, umsetzt und anschließend gegebenenfalls W und/oder R² unter Bildung der freien Carboxygruppen abspaltet, oder
b₂) eine Verbindung der unter b₁) genannten Formel IV mit einer Verbindung der allgemeinen Formel VI, worin R² die Bedeutung wie in Formel I hat, und mit einer Verbindung der allgemeinen Formel VII, 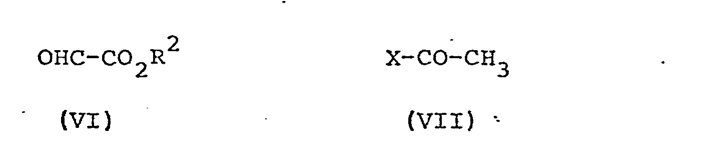 worin X die Bedeutung wie in Formel I hat, umsetzt und anschließend gegebenenfalls W und/oder R² unter Bildung der freien Carboxygruppen abspaltet, oder
c) daß man zur Herstellung von Verbindungen der Formel I, in der Y und Z jeweils Wasserstoff bedeuten , eine Verbindung der unter b₁) genannten Formel IV mit einer Verbindung der Formel VIII, 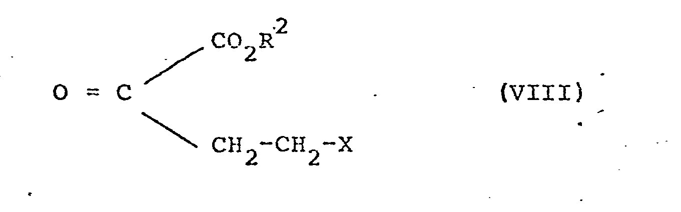 worin R² und X die Bedeutungen wie in Formel. I besitzen, umsetzt, die erhaltenen Schiff-Basen reduziert und anschließend gegebenenfalls W und/oder R² unter Bildung der freien Carboxygruppen abspaltet oder
d) daß man zur Herstellung von Verbindungen der Formel I, in der Y = Hydroxy und Z = Wasserstoff bedeuten , eine Verbindung der Formel I, in der Y und Z zusammen Sauerstoff bedeuten, mit einem komplexen Boranat oder Boran-Amin-Komplex reduziert.
die nach a) bis d) erhaltenen Verbindungen der Formel I, in welcher R für Wasserstoff steht, gegebenenfalls in Ester der Formel I, worin R (C₁ bis C₆)-Alkyl oder (C₇ bis C₉)-Aralkyl bedeutet, überführt und Verbindungen der Formel I gegebenenfalls in ihre physiologisch unbedenklichen Salze überführt.

2. Verfahren gemäß Anspruch 1, dadurch gekennzeichnet, daß Verbindungen der Formel I hergestellt werden, worin das C-Atom in Position 3 des bicyclischen Ringsystems sowie die mit einem Stern markierten C-Atome der Seitenkette jeweils S-Konfiguration aufweisen.

3. Verfahren gemäß einem der Ansprüche 1 oder 2, dadurch gekennzeichnet, daß Verbindungen der Formel I hergestellt werden, worin
n = 1
R = Wasserstoff oder (C₁ bis C₄)-Alkyl,
R¹ = Wasserstoff, (C₁ bis C₃)-Alkyl, die gegebenenfalls acylierte Seitenkette von Lysin, (C₂ oder C₃)-Alkenyl, Benzyl, die O-(C₁ bis C₆)-alkylierte Seitenkette von Tyrosin,, Phenethyl, 4-Amino-butyl oder Benzoylmethyl
R² = Wasserstoff, (C₁ bis C₄)-Alkyl oder Benzyl.
X = Cyclohexyl oder Phenyl, das durch (C₁ oder C₂)-Alkyl, (C₁ oder C₂)-Alkoxy, Hydroxy, Fluor, Chlor, Brom, Amino, (C₁ bis C₄)-Alkylamino, Di-(C₁ bis C₄)alkyl-amino, Nitro und/ oder Methylendioxy mono- oder disubstituiert und im Falle von Methoxy, trisubstituiert sein kann, bedeuten.

4. Verfahren gemäß einem der Ansprüche 1 bis 3 zur Herstellung einer Verbindung der Formel I zur Verwendung als Heilmittel.

5. Verfahren zur Herstellung eines Mittels enthaltend eine Verbindung gemäß einem der Ansprüche 1 bis 3 dadurch gekennzeichnet, daß man die Verbindung der Formel I in eine geeignete Darreichungsform bringt.

6. Verfahren zur Herstellung von Verbindungen der Formel III, in der W die in Anspruch 1 definierte Bedeutung hat, dadurch gekennzeichnet, daß man
a) Verbindungen der Formel IX, 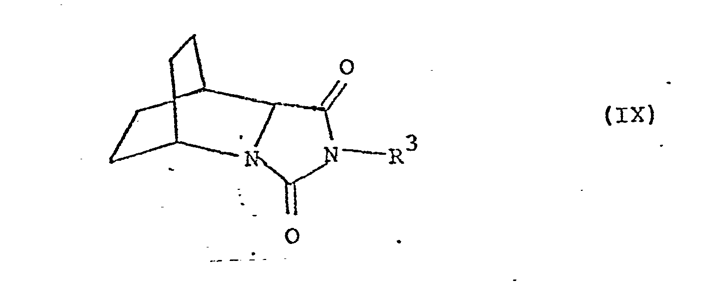 in welcher R³ einen (C₁-C₆)-Alkyl, Aryl-(C₁-C₃)-alkyl , Phenyl, 4-Methoxyphenyl- oder 4-Chlorphenylrest darstellt, mit einem Alkali- oder Erdalkalihydroxid, in Wasser oder Gemischen davon mit einem organischen Lösungsmittel verseifte und die erhaltene Aminosäure (III/W=Wasserstoff) gegebenenfalls nach üblichen Methoden der Aminosäürechemie verester oder
b) eine Verbindung der Formel X 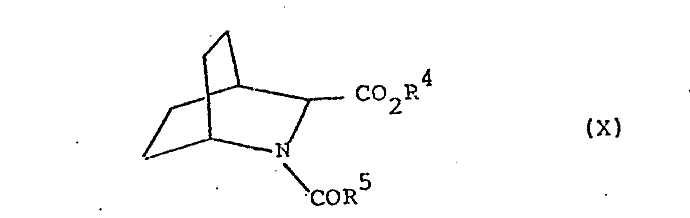 in welcher R⁴ einen (C₁ bis C₆)- oder Aryl-(C₁-C₃)-alkyl-, und R⁵ einen Phenyl-, Methyl-, (C₁ bis C )-Alkoxy- oder Aryl-(C₁ bis C₃)-alkoxy-Rest bedeuten unter den Bedingungen der Variante a) mit einem Alkali- oder Erdalkalihydroxid umsetzt und die erhaltene- Aminosäure (III, W = Wasserstoff) gegebenenfalls verestert, oder
c) eine Verbindung der Formel XI 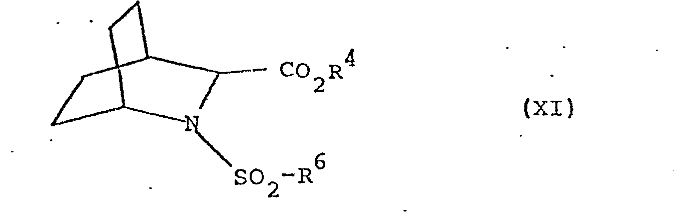 in welcher R⁴ die gleiche Bedeutung wie in FOrmel X besitzt und R⁶ einen Phenyl-, 4-Methylphenyl- oder 4-Chlorphenylrest darstellt, zunächst mit einem Alkali- oder Erdalkalimetall, in flüssigem Ammoniak bei -60° bis -10°C, und anschließend mit einem Alkali-oder Erdalkalihydroxid bei 20° bis 120°C, insbesondere 60 bis 100°C, umsetzt und gegebenenfalls verestert, oder die Reihenfolge der beschriebenen Reaktionsschritte umkehrt.

7. Verfahren zur Herstellung eines Mittels enthaltend eine Verbindung gemäß einem der Ansprüche 1 bis 3 in Kombination mit einem Diuretikum, dadurch gekennzeichnet, daß man die Verbindung der Formel I zusammen mit einem Diuretikum in eine geeignete Darreichungsform bringt.
